# EUROPEAN PATENT APPLICATION

(11) **EP 3 778 856 A1**
(43) Date of publication of application: **17.02.2021**
(21) Application number: 19775089.6
(22) Date of filing: 06.02.2019
(51) Int. Cl.: C12M 1/34, C12N 5/0783, C12Q 1/04

(54) **INFORMATION PROCESSING DEVICE, INFORMATION PROCESSING METHOD, AND PROGRAM**

(30) Priority: 29.03.2018 JP 2018063867
(71) Applicant: Sony Corporation, 108-0075 Tokyo (JP)
(72) Inventor: ABE, Tomoteru, Tokyo 108-0075 (JP); SASADA, Shiori, Tokyo 108-0075 (JP)
(74) Representative: D Young & Co LLP
(86) International application number: PCT/JP2019/004298
(87) International publication number: WO 2019/187673

(57) **Abstract**

To determine efficacy of an immunotherapeutic preparation for a patient quickly and easily.

An information processing device includes an image acquiring unit that acquires an image including mitochondria of immune cells collected from a subject, an analysis unit that analyzes a characteristic amount relating to activity of the mitochondria specified from the acquired image, and an output unit that outputs information regarding efficacy of an immunotherapeutic preparation for the subject on the basis of an analysis result of the analysis unit.

## Description

### TECHNICAL FIELD

The present disclosure relates to an information processing device, an information processing method, and a program.

### BACKGROUND ART

In recent years, an immunotherapeutic preparation has been attracting attention as a therapeutic agent for cancer, an autoimmune disease, or the like.

The immunotherapeutic preparation is a therapeutic agent that treats a disease by inducing, enhancing, or suppressing an immune response. Examples of the immunotherapeutic preparation include an immunosuppressive agent applied to an autoimmune disease and an allergic disease, a cancer immunotherapeutic agent used for cell immunotherapy or cytokine therapy, an immune checkpoint inhibitor used for antibody therapy, and the like.

For example, in recent years, development of the immune checkpoint inhibitor used for antibody therapy has progressed largely. The immune checkpoint inhibitor is, for example, a monoclonal antibody that targets a molecule involved in an immune checkpoint mechanism that controls activity of immune cells that attack cancer cells. The immune checkpoint inhibitor inhibits an immune checkpoint signal which cancer cells use in order to escape attacks from immune cells, thereby can release suppression of activity of the immune cells against the cancer cells, and can enhance an antitumor immune response of a human body.

For example, the following Patent Document 1 discloses a technique of treating cancer by administering a population of isolated natural killer cells, and an antibody that is specifically bonded to an immune checkpoint protein and antagonizes activity of the immune checkpoint protein.

### CITATION LIST

### PATENT DOCUMENT

Patent Document 1: Japanese Translation of PCT International Application Publication No. 2018-502114

### SUMMARY OF THE INVENTION

### PROBLEMS TO BE SOLVED BY THE INVENTION

However, many of these immunotherapeutic preparations are biopharmaceuticals containing protein as a main raw material, and therefore pharmaceutical cost thereof is extremely high. Furthermore, there is a difference in human immune function between individuals. In particular, an immunotherapeutic preparation such as an immune checkpoint inhibitor attacks cancer cells by activating autoimmunity of a patient, and therefore has a difference in efficacy between individuals. Therefore, it is not realistic to administer an immunotherapeutic preparation to all patients who can be therapeutic targets because of huge cost.

Therefore, it has been required to determine efficacy of an immunotherapeutic preparation for a patient before a full-scale treatment with the immunotherapeutic preparation is started.

### SOLUTIONS TO PROBLEMS

The present disclosure provides an information processing device including an image acquiring unit that acquires an image including mitochondria of immune cells collected from a subject, an analysis unit that analyzes a characteristic amount relating to activity of the mitochondria specified from the acquired image, and an output unit that outputs information regarding efficacy of an immunotherapeutic preparation for the subject on the basis of an analysis result of the analysis unit.

Furthermore, the present disclosure provides an information processing method for causing an arithmetic processing device to acquire an image including mitochondria of immune cells collected from a subject, to analyze a characteristic amount relating to activity of the mitochondria specified from the acquired image, and to output information regarding efficacy of an immunotherapeutic preparation for the subject on the basis of a result of the analysis.

Furthermore, the present disclosure provides a program that causes a computer to function as an image acquiring unit that acquires an image including mitochondria of immune cells collected from a subject, an analysis unit that analyzes a characteristic amount relating to activity of the mitochondria specified from the acquired image, and an output unit that outputs information regarding efficacy of an immunotherapeutic preparation for the subject on the basis of an analysis result of the analysis unit.

According to the present disclosure, from morphological characteristics or dynamic characteristics of mitochondria of immune cells of a subject to whom an immunotherapeutic preparation has been administered, or characteristics of fluorescence emitted from the mitochondria, it can be determined whether or not the immune cells of the subject have recovered antitumor activity by administration of the immunotherapeutic preparation. Since recovery of the antitumor activity of the immune cells occurs as a previous step of regression of cancer cells, doctors and the like can find efficacy of the immunotherapeutic preparation for the subject without waiting for the regression of cancer cells.

### EFFECTS OF THE INVENTION

As described above, according to the present disclosure, it is possible to determine efficacy of an immunotherapeutic preparation for a patient quickly and easily.

Note that the above effect is not necessarily limited. Any one of effects described in the present specification or another effect that can be grasped from the present specification may be exhibited together with the above effect or in place of the above effect.

### BRIEF DESCRIPTION OF DRAWINGS

Fig. 1 is a schematic diagram schematically illustrating a configuration example of an entire system including an information processing device according to an embodiment of the present disclosure.
Fig. 2 is a block diagram illustrating a configuration example of the information processing device according to the embodiment.
Fig. 3A is a model diagram of a fluorescence image of fission type mitochondria with reduced activity.
Fig. 3B is a model diagram of a fluorescence image of activated fusion type mitochondria.
Fig. 4 is a graph illustrating a result obtained by measuring the circularity of each of fission type mitochondria and fusion type mitochondria.
Fig. 5 is an explanatory diagram schematically illustrating an analysis method using a machine learning algorithm.
Fig. 6 is a graph in which a generation of machine learning and a correct answer ratio of an analysis result by machine learning are associated with each other.
Fig. 7 is a schematic diagram illustrating exchange of information by the information processing device.
Fig. 8 is a flowchart for explaining an operation example of the information processing device.
Fig. 9 is a block diagram illustrating an example of a hardware configuration of the information processing device.

### MODE FOR CARRYING OUT THE INVENTION

Preferable embodiments of the present disclosure will be described in detail below with reference to the accompanying drawings. Note that in the present specification and drawings, the same reference numerals are given to constituent elements having substantially the same functional configuration, and redundant explanation is omitted.

Note that the description will be made in the following order.
1. Technical background of the present disclosure
2. Configuration of entire system
3. Configuration of information processing device
4. Analysis method by analysis unit
4.1. First analysis method
4.2. Second analysis method
5. Operation of information processing device
6. Hardware configuration

### <1. Technical background of the present disclosure>

First, a technical background of an information processing device according to an embodiment of the present disclosure will be described. An object of the information processing device according to the present embodiment is to determine efficacy of an immunotherapeutic preparation for a subject quickly and easily.

Here, the immunotherapeutic preparation is a therapeutic agent that treats a disease by inducing, enhancing, or suppressing an immune response. Examples of the immunotherapeutic preparation include an immunosuppressive agent applied to an autoimmune disease and an allergic disease, a cancer immunotherapeutic agent used for cell immunotherapy or cytokine therapy, an immune checkpoint inhibitor used for antibody therapy, and the like. Hereinafter, description will be made particularly focusing on the immune checkpoint inhibitor.

The body of an organism has a mechanism that suppresses occurrence of an autoimmune disease and the like by suppressing an excessive immune reaction of T cells which are immune cells. Such a mechanism that negatively controls immunity is called an immune checkpoint mechanism.

By utilizing this immune checkpoint mechanism, cancer cells suppress or avoid attacks from immune cells and maintain their proliferative ability. The immune checkpoint inhibitor acts on the immune checkpoint mechanism which cancer cells utilize in order to suppress or avoid antitumor activity of immune cells, releases control by the immune checkpoint mechanism, and thereby activates attacks on cancer cells by immune cells. As a result, the immune checkpoint inhibitor can exhibit an antitumor effect.

Examples of the immune checkpoint inhibitor include a monoclonal antibody targeting CTLA-4 that suppresses activation of T cells by being bonded to T cell CD28 (for example, ipilimumab), a monoclonal antibody that targets T cell PD-1 and antagonizes PD-L1 expressed on surfaces of cancer cells in order to escape attacks from T cells (for example, nivolumab), and the like.

However, whether or not these immune checkpoint inhibitors are efficacious depends on what immune checkpoint mechanism cancer cells of a patient utilize to avoid or suppress attacks from immune cells. Therefore, a specific immune checkpoint inhibitor is not efficacious against some cancer cells in some cases.

As a method for determining efficacy of an immune checkpoint inhibitor, it is conceivable to detect whether or not a molecule targeted by the immune checkpoint inhibitor or a molecule that antagonizes the immune checkpoint inhibitor is expressed in cancer cells of a patient. For example, in a case of a PD-1 inhibitor such as nivolumab, it is conceivable to predict efficacy of the PD-1 inhibitor by detecting whether or not a PD-L1 molecule is expressed in cancer cells of a patient.

However, recent studies have revealed that it is difficult to sufficiently determine efficacy of an immune checkpoint inhibitor by such a method. Therefore, there has been a demand for a method capable of determining efficacy of an immune checkpoint inhibitor for a cancer patient with higher accuracy.

Meanwhile, recent studies have provided new finding about characteristics of cancer cells.

For example, a paper "Proc Natl Acad Sci USA, 2017 Jan 31, 114 (5), E761-E770" revealed that when a PD-1 inhibitor was administered to cancer-transplanted mice, there was a difference in oxygen consumption rate (OCR) of CD8 + T cells between a group in which the PD-1 inhibitor was efficacious and cancer regression was observed and a group in which the PD-1 inhibitor was not efficacious. Specifically, this paper reported that the OCR of CD8 + T cells collected from mice of the group in which the PD-1 inhibitor was efficacious and cancer regression was observed was significantly increased as compared with the OCR of CD8 + T cells collected from mice of the group in which the PD-1 inhibitor was not efficacious.

It is considered that this indicates that in cancer against which the PD-1 inhibitor is efficacious, addition of the PD-1 inhibitor activates an effector function of immune cells suppressed by PD-L1 of cancer cells, and therefore the immune cells are ready to attack the cancer cells. According to this finding, by measuring the state of CD8 + T cells of a cancer patient to whom the PD-1 inhibitor has been administered, efficacy of an immune checkpoint inhibitor targeting PD-1 for the cancer patient may be able to be determined.

Furthermore, a paper "Cell, 2016 Jun 30, 166 (1), 63-76" has revealed that the forms of mitochondria of T cells change depending on the states of differentiation and activation of the T cells. That is, it has been revealed that mitochondria, which are important organelles that regulate the role and state of cells by performing energy metabolism to synthesize adenosine triphosphate (ATP), dynamically change their forms depending on an active state of the cells. Moreover, this paper suggests that the change in the forms of mitochondria of T cells is closely related to control of activation and quiescence of an effector function on cancer cells.

The present inventors examined the findings of the above studies intensively, and thereby have found that in a cancer patient to whom the PD-1 inhibitor is efficacious, CD8 + T cells are activated by administration of the PD-1 inhibitor, and a characteristic change may occur in the forms of mitochondria of CD8 + T cells. As a result, the present inventors have got an idea of the technology according to the present disclosure.

The technology according to the present disclosure determines efficacy of an immunotherapeutic preparation for a subject by analyzing morphological characteristics or dynamic characteristics of mitochondria of immune cells collected from the subject. More specifically, the technology according to the present disclosure determines efficacy of a PD-1 inhibitor type immune checkpoint inhibitor for a cancer patient on the basis of morphological characteristics or dynamic characteristics of mitochondria of CD8 + T cells collected from the cancer patient.

In particular, in the technology according to the present disclosure, in a case where morphological characteristics or dynamic characteristics of mitochondria of immune cells collected from a subject are analyzed, efficacy of an immunotherapeutic preparation can be determined by observing each cell unit. This makes it possible to determine efficacy of an immunotherapeutic preparation without collecting a large amount of immune cells from a subject, and therefore makes it possible to reduce a burden on the subject.

Note that the subject in the technology according to the present disclosure indicates a subject from which immune cells can be collected. Examples of the subject include a cancer patient, a healthy person, a mouse for animal experiments, and the like, but are not limited thereto. The subject may be either a human or an animal.

Hereinafter, an information processing device that implements the technology according to the present disclosure will be specifically described.

### <2. Configuration of entire system>

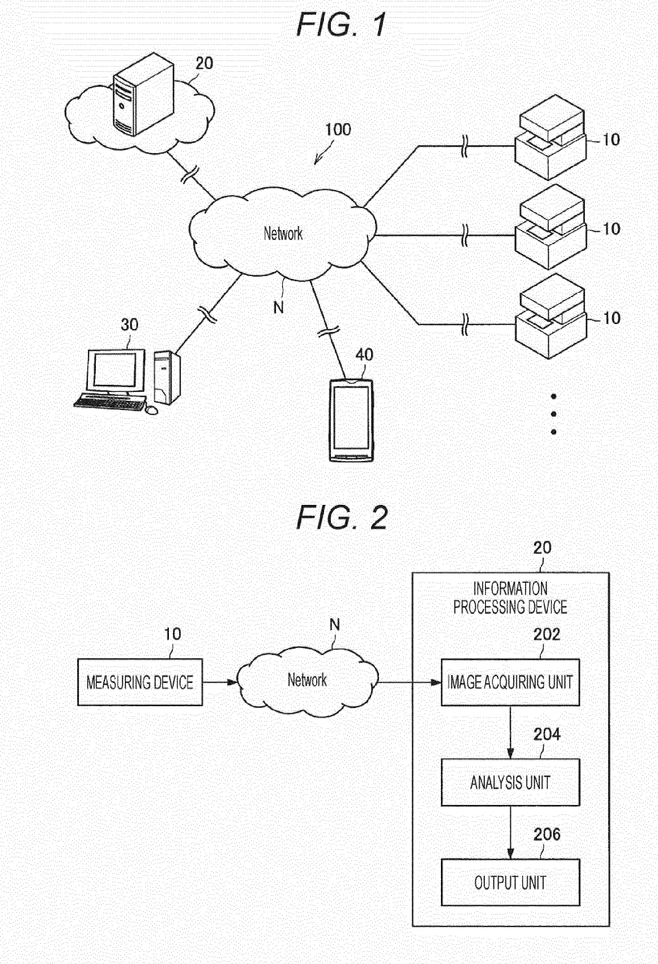

First, a configuration of a system 100 including an information processing device according to an embodiment of the present disclosure will be described with reference to Fig. 1. Fig. 1 is a schematic diagram schematically illustrating a configuration example of the system 100 including the information processing device according to the present embodiment.

As illustrated in Fig. 1, the system 100 according to the present embodiment includes a measuring device 10, an information processing device 20, and terminal devices 30 and 40. Note that the measuring device 10, the information processing device 20, and the terminal devices 30 and 40 are connected to each other via a network N so as to be able to communicate with each other. The network N may be, for example, the Internet, a mobile communication network, a local area network, or the like, or may be a combination of a plurality of types of these networks.

The measuring device 10 can observe mitochondria of immune cells collected from a subject. For example, the measuring device 10 may be a single cell analysis device that captures one cell in each of a large number of microwells arranged on a plane and observes the form of each cell individually to analyze characteristics of each cell, may be a cell sorter or a flow cytometer capable of sorting specific immune cells by using a surface antigen such as CD8 as a mark, or may be a fluorescence microscope or a confocal laser microscope capable of observing mitochondria of sorted immune cells or mitochondria stained by immunohistochemical staining (hereinafter collectively referred to as mitochondria of immune cells).

The information processing device 20 determines efficacy of an immunotherapeutic preparation for a subject by acquiring an image and the like of mitochondria of immune cells observed by the measuring device 10 and analyzing morphological characteristics or dynamic characteristics of mitochondria of the immune cells. The information processing device 20 may be, for example, a server capable of processing a large amount of data at high speed.

The terminal devices 30 and 40 are information processing devices that output results of efficacy of an immunotherapeutic preparation determined by the information processing device 20. For example, each of the terminal devices 30 and 40 may be a computer, a laptop, a smartphone, or a tablet terminal including a display unit that displays an output determination result with images, characters, and the like.

The system 100 including the information processing device 20 according to the present embodiment first acquires an image and the like obtained by imaging mitochondria of immune cells of a subject from each of the measuring devices 10 disposed in a hospital, a clinic, or a research institute. Thereafter, the system 100 according to the present embodiment outputs efficacy of an immunotherapeutic preparation determined using the image to the terminal devices 30 and 40 to feed back a determination result to a doctor, a researcher, and the like. The information processing device 20 according to the present embodiment acquires an image of mitochondria of immune cells of a cancer patient via the network N, feeds back a determination result using the acquired image of mitochondria, and can thereby handle a larger amount of data.

### <3. Configuration of information processing device>

Next, a configuration example of the information processing device according to the present embodiment will be described with reference to Fig. 2. Fig. 2 is a block diagram illustrating a configuration example of the information processing device according to the present embodiment.

As illustrated in Fig. 2, the information processing device 20 includes an image acquiring unit 202, an analysis unit 204, and an output unit 206.

The image acquiring unit 202 is an input interface that acquires an image obtained by imaging mitochondria of immune cells from the measuring device 10 via the network N. The image acquiring unit 202 may be, for example, an interface capable of communicating with the measuring device 10 directly or via the network N, or an interface capable of wired or wireless communication with a gateway terminal connected to the network N.

Here, an image acquired by the image acquiring unit 202 is an image obtained by imaging mitochondria of immune cells collected from a subject to whom an immunotherapeutic preparation has been administered or a subject to whom no immunotherapeutic preparation has been administered. Specifically, the image acquired by the image acquiring unit 202 may be an image obtained by fluorescently staining CD8 + T cells among lymphocytes collected from lymph nodes and the like of a subject who is a cancer patient in a mitochondria-specific manner. Such an image can be acquired by staining mitochondria of CD8 + T cells in an organelle-specific manner and then imaging the CD8 + T cells with a fluorescence microscope, a confocal laser microscope, or the like. The CD8 + T cells can be acquired, for example, by sorting the CD8 + T cells from a lymph fluid, blood, and the like of a subject using a cell sorter and the like by using a surface antigen such as CD8 as a mark. Furthermore, the image acquired by the image acquiring unit 202 may be an image obtained by imaging mitochondria of immune cells to which an immunotherapeutic preparation has been administered while the concentration of the preparation is gradually changed after the immune cells are collected from a subject.

However, the image acquired by the image acquiring unit 202 may be an image obtained by imaging mitochondria of immune cells other than CD8 + T cells. For example, the image acquiring unit 202 may acquire an image obtained by imaging mitochondria of other lymphocytes such as B cells or NK cells, or may acquire an image obtained by imaging mitochondria of immune cells other than lymphocytes.

The image acquiring unit 202 may acquire a plurality of images. There is a difference between individual immune cells even if the immune cells are collected from the same subject. Therefore, the information processing device 20 can determine efficacy of an immunotherapeutic preparation with higher accuracy by determining the efficacy using a plurality of images obtained by imaging mitochondria of immune cells collected from the same subject.

The image acquired by the image acquiring unit 202 may be an image obtained by simultaneously imaging mitochondria of a plurality of types of immune cells. For example, the image acquiring unit 202 may acquire an image obtained by simultaneously imaging mitochondria of CD8 + T cells and B cells.

The image acquired by the image acquiring unit 202 may be an image obtained by simultaneously imaging immune cells and mitochondria. Specifically, the image acquired by the image acquiring unit 202 may be an image obtained by simultaneously fluorescently staining cell membranes of CD8 + T cells and mitochondria. As a result, the information processing device 20 can observe localization of mitochondria and the fluorescence amount thereof in one cell and can also acquire information regarding the localization of mitochondria and the fluorescence amount thereof.

The image acquired by the image acquiring unit 202 may be a still image, a moving image, or a time-lapse image. For example, in a case where the information processing device 20 analyzes morphological characteristics of mitochondria, the image acquired by the image acquiring unit 202 is preferably a still image. Meanwhile, in a case where the information processing device 20 analyzes dynamic characteristics of mitochondria, the image acquired by the image acquiring unit 202 is preferably a moving image or a time-lapse image.

However, the image acquired by the image acquiring unit 202 is not limited to the above image as long as the shape and the like of mitochondria of immune cells can be confirmed, and any image can be used. For example, the image acquired by the image acquiring unit 202 may be an image obtained by observing immune cells with an optical microscope, an electron microscope (for example, SEM), or the like.

The analysis unit 204 analyzes morphological characteristics or dynamic characteristics of mitochondria or characteristics of fluorescence emitted from mitochondria in the image acquired by the image acquiring unit 202.

As described above, it has been reported that mitochondria become a fusion type having an elongated filamentous shape by fusing with each other in a state where ATP synthesis is active, and become a fission type having an elliptical shape by being separated from each other in a state where ATP synthesis is calmed. It is considered that this is because the fusion type mitochondria are deformed into an elongated filamentous shape such that an interval between inner membranes (or cristae) is narrowed and ATP synthesis is performed efficiently. Meanwhile, it is considered that the fission type mitochondria are deformed into an elliptical shape such that an interval between inner membranes (or cristae) is loosened and efficiency of ATP synthesis is reduced.

Therefore, it is considered that activity of mitochondria can be determined by analyzing morphological characteristics such as the outer shapes of mitochondria, the sizes thereof, or the internal structures thereof. Furthermore, in a case where the shapes of mitochondria change, behaviors of mitochondria also change similarly. Therefore, it is considered that activity of mitochondria can also be determined by analyzing dynamic characteristics such as vibration of mitochondria, movement thereof, or a shape change thereof.

Specifically, the analysis unit 204 may extract a predetermined characteristic amount from mitochondria in the image acquired by the image acquiring unit 202 and analyze activity of the mitochondria on the basis of the extracted characteristic amount. Alternatively, the analysis of activity of mitochondria may be analyzing whether the mitochondria are a fusion type or a fission type. The predetermined characteristic amount may be any characteristic amount as long as being able to distinguish activity of mitochondria, and may be, for example, the circularity of mitochondria, the outer peripheral length thereof, or the occupation area thereof with respect to immune cells. The analysis unit 204 may analyze activity of mitochondria in the image on the basis of whether or not the predetermined characteristic amount extracted from the mitochondria exceeds a threshold.

Moreover, the analysis unit 204 may analyze activity of mitochondria on the basis of a characteristic amount of fluorescence emitted from the mitochondria. For example, the analysis unit 204 may analyze activity of mitochondria on the basis of information regarding the saturation of fluorescence of the mitochondria, the brightness thereof, or the plane distribution thereof. Note that examples of the above-described fluorescence emitted from mitochondria include autofluorescence of mitochondria and fluorescence derived from fluorescent molecules that label mitochondria.

Alternatively, the analysis unit 204 may analyze, by a machine learning algorithm, to which of mitochondria of immune cells collected from a patient to whom an immunotherapeutic preparation is efficacious and mitochondria of immune cells collected from a patient to whom the immunotherapeutic preparation is not efficacious, mitochondria in the image acquired by the image acquiring unit 202 is closer. Specifically, the analysis unit 204 may analyze, by a machine learning algorithm, the degree of agreement (or probability of appropriateness) between mitochondria in the image acquired by the image acquiring unit 202 and the above-described mitochondria of immune cells collected from a patient to whom an immunotherapeutic preparation is efficacious.

The machine learning algorithm used by the analysis unit 204 is not particularly limited, and may be any algorithm of supervised learning, unsupervised learning, semi-supervised learning, and reinforcement learning. For example, the machine learning algorithm may be an algorithm using a neural network such as recurrent neural network (RNN), cnventional neural network (CNN), or multilayer perceptron (MLP), a support vector machine, clustering, or the like. More specifically, the machine learning algorithm may be an algorithm using a multilayer neural network (so-called deep learning).

Note that a method for analyzing morphological characteristics or dynamic characteristics of mitochondria or characteristics of fluorescence emitted from mitochondria in an image by the analysis unit 204 will be described in detail later.

The output unit 206 outputs information regarding efficacy of an immunotherapeutic preparation for a subject on the basis of an analysis result of the analysis unit 204. Specifically, the output unit 206 first determines whether or not the administered immunotherapeutic preparation is efficacious for a subject on the basis of the analysis result of the analysis unit 204. Specifically, the output unit 206 determines activity of mitochondria of immune cells of a subject on the basis of the analysis result of the analysis unit 204, and thereby determines whether or not the immune cells of the subject are activated by the immunotherapeutic preparation. As a result, the output unit 206 can determine whether or not the administered immunotherapeutic preparation is efficacious for the subject. Thereafter, the output unit 206 outputs information regarding efficacy of the immunotherapeutic preparation for the subject on the basis of a determination result. For example, as the information regarding efficacy, the output unit 206 may output a result obtained by determining whether or not the immunotherapeutic preparation is efficacious for the subject, or may output a result obtained by determining the degree of efficacy of the immunotherapeutic preparation for the subject.

For example, the output unit 206 may determine activity of mitochondria in each of images obtained by imaging mitochondria of a plurality of immune cells, and may determine efficacy of the immunotherapeutic preparation comprehensively on the basis of results obtained by determining mitochondria in the plurality of images. There is a difference between individual immune cells even if the immune cells are collected from the same subject. Therefore, the output unit 206 can determine efficacy of the immunotherapeutic preparation with higher accuracy by using results obtained by determining mitochondria of a plurality of immune cells.

More specifically, in a case where the ratio of immune cells having fusion type mitochondria among immune cells collected from a subject to whom the immunotherapeutic preparation has been administered is equal to or more than a threshold, the output unit 206 may determine that the immunotherapeutic preparation is efficacious for the subject. Alternatively, in a case where the ratio of immune cells having fusion type mitochondria increases after administration of the immunotherapeutic preparation as compared with that before the administration, the output unit 206 may determine that the immunotherapeutic preparation is efficacious for the subject.

Alternatively, in a case where the ratio or amount of mitochondria determined to be activated in immune cells collected from a subject to whom the immunotherapeutic preparation has been administered exceeds a threshold, the output unit 206 may determine that the immunotherapeutic preparation is efficacious for the subject.

Note that the output unit 206 may include an output interface that outputs information regarding efficacy of an immunotherapeutic preparation for a subject to the terminal devices 30 and 40 and the like via the network N. The output interface included in the output unit 206 may be, for example, an interface capable of communicating with the terminal devices 30 and 40 directly or via the network N, or an interface capable of wired or wireless communication with a gateway terminal connected to the network N.

According to the above configuration, the information processing device 20 according to the present embodiment can determine whether or not immune cells are activated by an immunotherapeutic preparation from an image of immune cells without waiting for regression of cancer cells due to antitumor activity of the immune cells. This makes it possible for the information processing device 20 to determine efficacy of an immunotherapeutic preparation for a subject who is a cancer patient quickly and easily.

### <4. Analysis method by analysis unit>

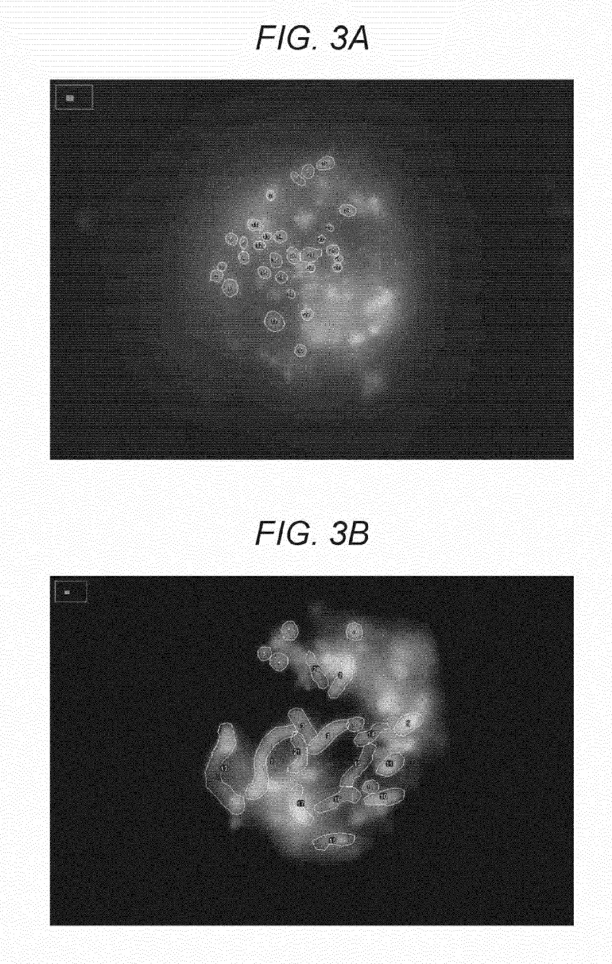

Subsequently, a method for analyzing morphological characteristics or dynamic characteristics of mitochondria by the analysis unit 204 will be described in detail with reference to Figs. 3A to 6.

By performing the following model experiments, immune cells having characteristics corresponding to each of fission type mitochondria and fusion type mitochondria were prepared.

Specifically, in a case where an uncoupling agent that eliminates a membrane potential of mitochondria is allowed to act on cells, the state of mitochondria can be converted into a fission type. Therefore, carbonyl cyanide m-chlorophenyl hydrazone (CCCP), which is an uncoupling agent, was allowed to act on Jurkat cells, which are cell lines derived from T cells, and CCCP was removed by washing. Thereafter, recovery culture was performed for several hours. At this time, the cells were divided into two cell groups. One of the cell groups was cultured in the presence of cycloheximide, which is a protein synthesis inhibitor, to maintain a morphological change of mitochondria by CCCP. The other cell group was cultured in the absence of cycloheximide to recover a morphological change of mitochondria by CCCP. Thereafter, mitochondria were stained with a chemical that specifically stains mitochondria, and a fluorescence image of mitochondria was imaged with a fluorescence microscope. Results thereof are schematically illustrated in Figs. 3A and 3B.

The fluorescence image of mitochondria schematically illustrated in Fig. 3A is a model of a fluorescence image of fission-type mitochondria with reduced activity. Specifically, in the mitochondria in the fluorescence image illustrated in Fig. 3A, a membrane potential is eliminated by an uncoupling agent, and the fission type state is maintained by cycloheximide. Therefore, the mitochondria in the fluorescence image illustrated in Fig. 3A is a model of mitochondria with reduced ATP synthesis activity, and is a model of mitochondria of immune cells inactivated by a suppressive effect of immune activity by cancer cells. As illustrated in Fig. 3A, the fission type mitochondria have outer shapes degenerated into elliptical shapes, and the mitochondria are separated from each other.

Meanwhile, the fluorescence image of mitochondria schematically illustrated in Fig. 3B is a model of a fluorescence image of activated fusion type mitochondria. Specifically, the mitochondria in the fluorescence image illustrated in Fig. 3B are subjected to recovery culture in the absence of cycloheximide. Therefore, the mitochondria in the fluorescence image illustrated in Fig. 3B is a model of mitochondria in which the ATP synthesis activity has returned to its original activity, and is a model of mitochondria of immune cells in which mitochondria are activated due to favorable efficacy of an immunotherapeutic preparation and antitumor activity has been regained. As illustrated in Fig. 3B, the fusion type mitochondria each have a thread-like outer shape, and the mitochondria are intertwined with each other.

As a method for analyzing the forms of mitochondria of immune cells as illustrated in Figs. 3A and 3B, the analysis unit 204 can use the following first or second analysis method.

### (4.1. First analysis method)

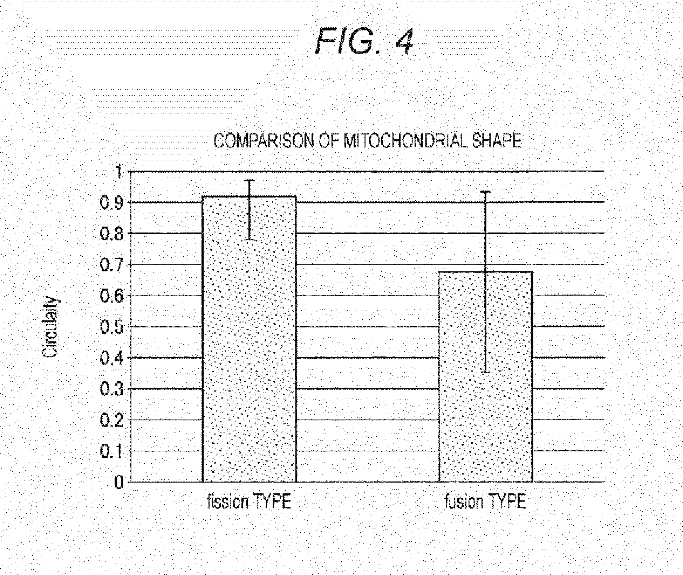

First, the first analysis method by the analysis unit 204 will be described with reference to Fig. 4. Fig. 4 is a graph illustrating a result obtained by measuring the circularity of each of fission type mitochondria and fusion type mitochondria.

In the first analysis method, the analysis unit 204 extracts a predetermined characteristic amount from mitochondria. As a result, the output unit 206 determines whether or not mitochondria are activated using the predetermined characteristic amount as an index.

Specifically, the analysis unit 204 may determine the outer shapes of mitochondria by image analysis, and then may extract a characteristic amount serving as an index capable of distinguishing fission type mitochondria and fusion type mitochondria from each other from the outer shapes of mitochondria. Examples of such an index include morphological characteristics such as the circularity of mitochondria, the roundness thereof, outer peripheral length thereof, or the occupied area thereof in immune cells.

For example, Fig. 4 illustrates results obtained by calculating a circularity represented by "4π × (area)/outer peripheral length²" in each of fission type mitochondria and fusion type mitochondria obtained in the model experiments illustrated in Figs. 3A and 3B in in a histogram. Note that as the circularity represented by "4π × (area)/outer peripheral length²" is closer to 1, the shape is closer to a perfect circle. As illustrated in Fig. 4, the fission type mitochondria have significantly higher circularity and outer shapes closer to perfect circles than the fusion type mitochondria. Therefore, by setting an appropriate threshold for the circularity of the outer shapes of mitochondria, it is possible to distinguish the fission type mitochondria and the fusion type mitochondria from each other.

Furthermore, in a case where morphological characteristics of mitochondria are different, dynamic behavior of mitochondria is considered to be different similarly. Therefore, the analysis unit 204 can also distinguish the fission type mitochondria and the fusion type mitochondria from each other by extracting a characteristic amount relating to dynamic characteristics such as vibration of mitochondria, movement thereof, or a shape change thereof, and setting a threshold on the extracted characteristic amount.

### (4.2. Second method)

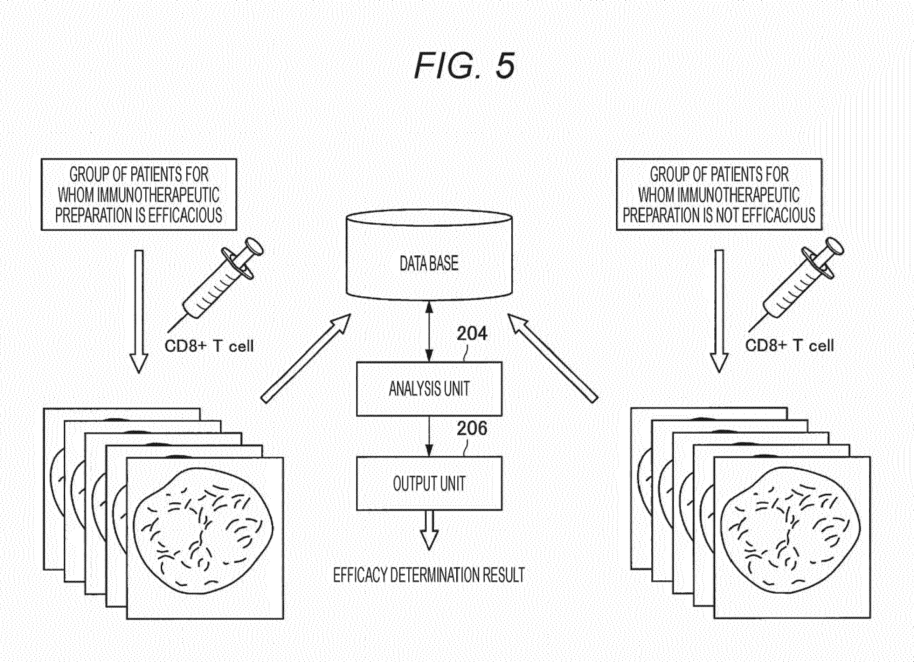

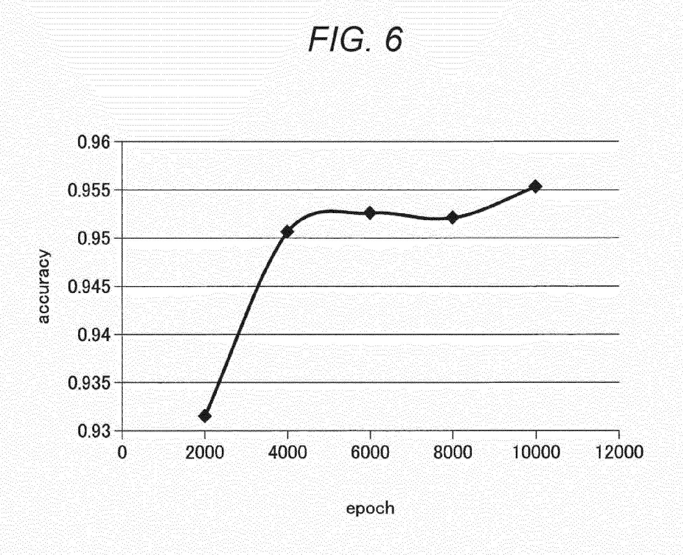

Next, the second analysis method by the analysis unit 204 will be described with reference to Figs. 5 and 6. Fig. 5 is an explanatory diagram schematically illustrating an analysis method using a machine learning algorithm. Fig. 6 is a graph in which a generation of machine learning and a correct answer ratio of an analysis result by machine learning are associated with each other.

In the second analysis method, the analysis unit 204 performs machine learning in advance using an image obtained by imaging mitochondria of immune cells of a cancer patient for whom an immunotherapeutic preparation is efficacious, and an image obtained by imaging mitochondria of immune cells of a cancer patient for whom the immunotherapeutic preparation is not efficacious. The analysis unit 204 can analyze, by an algorithm obtained by the machine learning, to which of mitochondria of a patient for whom an immunotherapeutic preparation is efficacious and mitochondria of a patient for whom the immunotherapeutic preparation is not efficacious, mitochondria in the acquired image is closer. As a result, the output unit 206 determines whether or not the immunotherapeutic preparation is efficacious for the subject on the basis of the analysis result by the analysis unit 204.

For example, Fig. 6 illustrates a correct answer ratio of an analysis result by an algorithm obtained by performing machine learning using 50 images of mitochondria of immune cells collected from a patient for whom an immunotherapeutic preparation is efficacious and 50 images of mitochondria of immune cells collected from a patient for whom the immunotherapeutic preparation is not efficacious. Specifically, 50 images were enlarged, reduced, rotated, or partially cut out, for example. A parameter not directly involved in classification determination of mitochondria was changed to regenerate an image, the amount of teacher data was thereby increased, and supervised learning was performed. It is considered that such deep learning machine learning using teacher data in which an image is randomly processed to increase the data amount is suitable for solving a simple classification problem.

As illustrated in Fig. 6, as a result of performing a total of five generations in which one generation (one epoch) included 2000 trials by machine learning using the deep learning described above, it was found that a high correct answer ratio of 95.5% or more was obtained for 22 test images.

It may be difficult to quantify the forms of mitochondria of immune cells with known shape parameters and the like. Furthermore, there may be a possibility that the forms of mitochondria of immune cells collected from a patient for whom an immunotherapeutic preparation is efficacious and the forms of mitochondria of immune cells collected from a patient for whom an immunotherapeutic preparation is not efficacious are not clearly distinguished from each other. Therefore, in a case where a machine learning algorithm capable of classifying the forms of mitochondria of immune cells without being bound by known shape parameters is used, the analysis unit 204 can analyze the forms of mitochondria with higher accuracy.

In addition, mitochondria of immune cells activated by an immunotherapeutic preparation may have an unknown morphological change as compared with mitochondria of immune cells inactivated by cancer cells. By using the machine learning algorithm, the analysis unit 204 can perform morphological analysis of mitochondria in consideration of an unknown morphological change.

### <5. Operation of information processing device>

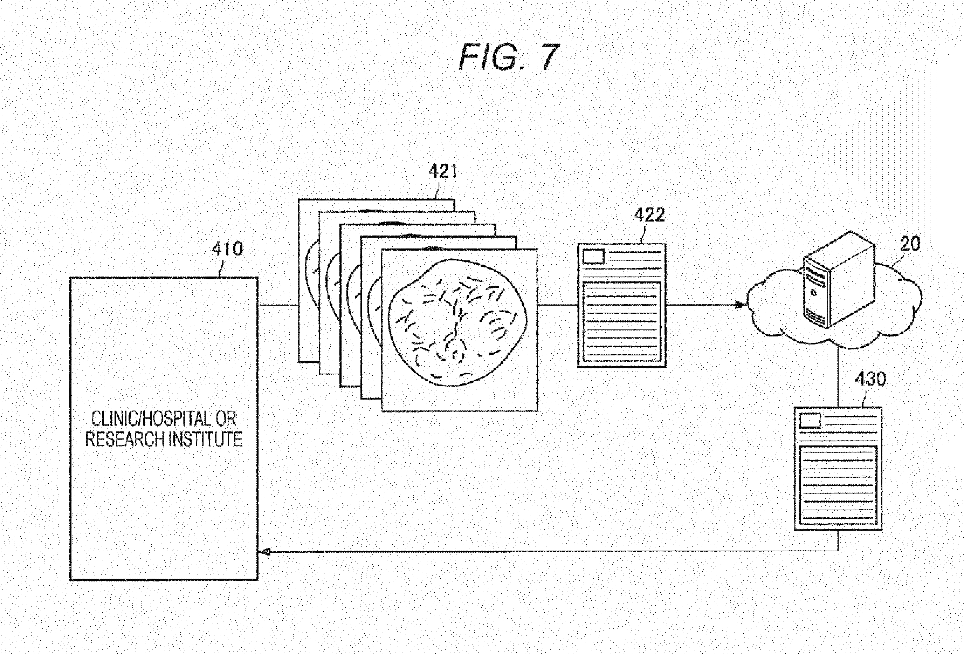

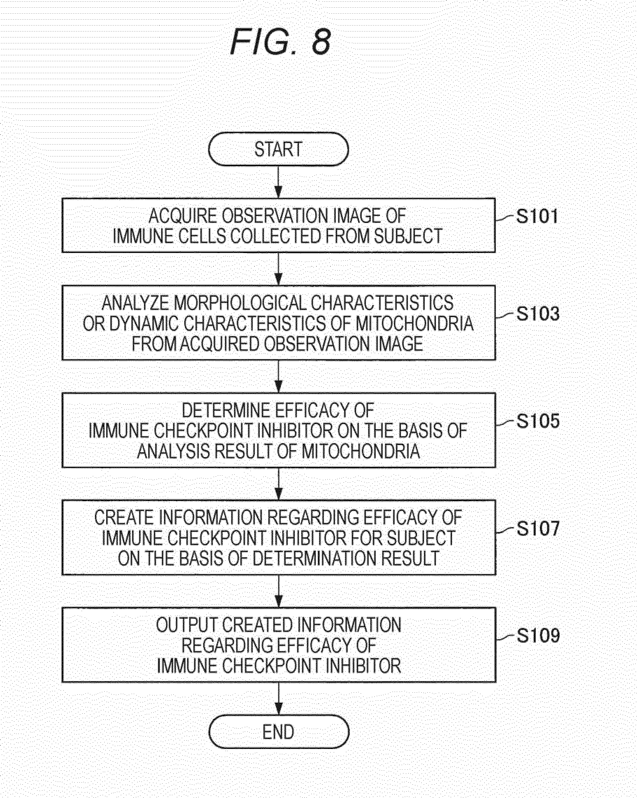

Next, an operation example of the information processing device 20 according to the present embodiment will be described with reference to Figs. 7 and 8. Fig. 7 is a schematic diagram illustrating exchange of information by the information processing device 20 according to the present embodiment. Fig. 8 is a flowchart for explaining an operation example of the information processing device 20 according to the present embodiment.

First, an operation example of the system 100 including the information processing device 20 will be schematically described with reference to Fig. 7.

As illustrated in Fig. 7, for example, an image 421 of immune cells of a cancer patient to whom an immunotherapeutic preparation has been administered is transmitted from a clinic/hospital or research institute 410 that treats a cancer patient to the information processing device 20.

Here, the image 421 of immune cells is an image obtained by imaging mitochondria. The image 421 of immune cells may be, for example, an image obtained by fluorescently staining CD8 + T cells in a mitochondria-specific manner and observing and imaging the CD8 + T cells with a fluorescence microscope. Furthermore, in addition to the image 421 of immune cells, information 422 regarding the cancer patient including attribute data, clinical data, and the like of the cancer patient may be transmitted from the clinic/hospital or research institute 410 to the information processing device 20.

The information processing device 20 determines the degree of activity of immune cells by analyzing the acquired image 421 of immune cells obtained by imaging mitochondria by the method described above, and determines efficacy of the immunotherapeutic preparation for the cancer patient. Subsequently, the information processing device 20 transmits information 430 regarding efficacy of the immunotherapeutic preparation for the cancer patient to the clinic/hospital or research institute 410 as a determination report on the basis of the determination result.

As a result, the clinic/hospital or research institute 410 can be notified of efficacy of the immunotherapeutic preparation for the cancer patient before starting a full-scale treatment of the cancer patient with the immunotherapeutic preparation. Therefore, the clinic/hospital or research institute 410 can refer to efficacy of an immunotherapeutic preparation as an index when administering the immunotherapeutic preparation to a cancer patient, and therefore can select a more appropriate treatment method for each cancer patient.

Subsequently, an operation example of the information processing device 20 will be specifically described with reference to Fig. 8.

As illustrated in Fig. 8, first, the information processing device 20 causes the image acquiring unit 202 to acquire an observation image of immune cells collected from a subject (S101). The observation image of immune cells may be, for example, a fluorescence observation image of CD8 + T cells in which mitochondria are specifically fluorescently stained.

Next, the information processing device 20 causes the analysis unit 204 to analyze morphological characteristics or dynamic characteristics of mitochondria from the acquired observation image (S103). The analysis unit 204 may, for example, analyze the state of mitochondria of the observation image by extracting a predetermined morphological characteristic amount or dynamic characteristic amount from the observation image, or may analyze the state of mitochondria of the observation image using a machine learning algorithm.

Subsequently, the information processing device 20 causes the output unit 206 to determine efficacy of the immunotherapeutic preparation on the basis of the analysis result of mitochondria of the observation image (S105). For example, the output unit 206 may determine that the immunotherapeutic preparation is efficacious in a case where the ratio of immune cells containing activated mitochondria among immune cells imaged in the observation image is equal to or higher than a threshold. Alternatively, the output unit 206 may determine efficacy of the immunotherapeutic preparation with an efficacy index on the basis of the ratio or number of immune cells containing active mitochondria.

Thereafter, the information processing device 20 creates information regarding efficacy of the immunotherapeutic preparation on the basis of the determination result (S107), and outputs the created information (S109). Here, for example, the information regarding efficacy of the immunotherapeutic preparation output by the information processing device 20 may indicate whether or not the immunotherapeutic preparation is efficacious, or may indicate efficacy of the immunotherapeutic preparation with an efficacy index.

According to the above operation, the information processing device 20 can determine efficacy of an immunotherapeutic preparation for a subject who is a cancer patient quickly and easily, and can support selection of a treatment method suitable for the subject.

### <6. Hardware configuration>

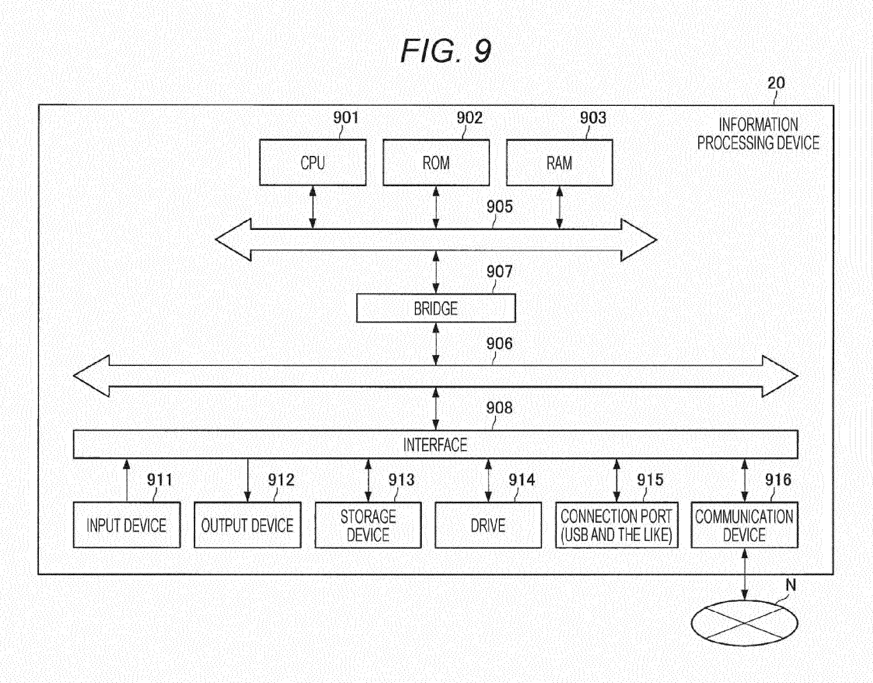

Subsequently, a hardware configuration of the information processing device 20 according to the present embodiment will be described with reference to Fig. 9. Fig. 9 is a block diagram illustrating a hardware configuration example of the information processing device 20 according to the present embodiment.

As illustrated in Fig. 9, the information processing device 20 includes a central processing unit (CPU) 901, a read only memory (ROM) 902, a random access memory (RAM) 903, a bridge 907, internal buses 905 and 906, an interface 908, an input device 911, an output device 912, a storage device 913, a drive 914, a connection port 915, and a communication device 916.

The CPU 901 functions as an arithmetic processing device and a control device, and controls overall operation of the information processing device 20 according to various programs stored in the ROM 902 and the like. The ROM 902 stores a program and an arithmetic parameter used by the CPU 901. The RAM 903 temporarily stores a program used in execution of the CPU 901, a parameter that changes appropriately during the execution, and the like. For example, the CPU 901 may execute some functions of the analysis unit 204 and the output unit 206.

The CPU 901, ROM 902, and RAM 903 are connected to each other by the bridge 907, the internal buses 905 and 906, and the like. Furthermore, the CPU 901, the ROM 902, and the RAM 903 are also connected to the input device 911, the output device 912, the storage device 913, the drive 914, the connection port 915, and the communication device 916 via the interface 908.

The input device 911 includes an input device that inputs information, such as a touch panel, a keyboard, a mouse, a button, a microphone, a switch, or a lever. Furthermore, the input device 911 also includes, for example, an input control circuit for generating an input signal on the basis of the input information and outputting the input signal to the CPU 901.

The output device 912 includes, for example, a display device such as a cathode ray tube (CRT) display device, a liquid crystal display device, or an organic electroluminescence (EL) display device. Moreover, the output device 912 may include an audio output device such as a speaker or a headphone.

The storage device 913 is a storage device for storing data of the information processing device 20. The storage device 913 may include a storage medium, a storage device that stores data in the storage medium, a reading device that reads data from the storage medium, and a deletion device that deletes the stored data.

The drive 914 is a reader/writer for a storage medium, and is built in or externally attached to the information processing device 20. For example, the drive 914 reads information stored in a removable storage medium such as a mounted magnetic disk, optical disc, magneto-optical disk, or semiconductor memory, and outputs the information to the RAM 903. The drive 914 can also write information to a removable storage medium.

The connection port 915 is a connection interface constituted, for example, by a connection port for connecting an external connection device such as a universal serial bus (USB) port, an Ethernet (registered trademark) port, an IEEE 802.11 standard port, or an optical audio terminal.

The communication device 916 is a communication interface constituted, for example, by a communication device for connection to the network 920, or the like. Furthermore, the communication device 916 may be a wired or wireless LAN communication device or a cable communication device that performs wired cable communication. The communication device 916 and the connection port 915 may execute, for example, some functions of the image acquiring unit 202 and the output unit 206.

Note that a computer program for causing hardware such as the CPU, the ROM, or the RAM built in the information processing device 20 to exhibit equivalent functions to the configurations of the above-described information processing device according to the present embodiment can also be created. Furthermore, a storage medium storing the computer program can also be provided.

Hitherto, preferable embodiments of the present disclosure have been described in detail with reference to the accompanying drawings, but the technical scope of the present disclosure is not limited to such examples. It is obvious that a person having ordinary knowledge in the technical field to which the present disclosure belongs can conceive of various change examples and modification examples within a range of the technical idea described in the claims, and it is understood that these change examples and modification examples are naturally within the technical scope of the present disclosure.

Furthermore, the effects described in the present specification are merely illustrative or exemplary, and are not limiting. That is, the technology according to the present disclosure can exhibit another effect obvious to those skilled in the art from the description of the present specification together with the above effects or in place of the above effects.

Note that the following configurations are also within the technical scope of the present disclosure.
(1)
   An information processing device including:
   an image acquiring unit that acquires an image including mitochondria of immune cells collected from a subject;
   an analysis unit that analyzes a characteristic amount relating to activity of the mitochondria specified from the acquired image; and
   an output unit that outputs information regarding efficacy of an immunotherapeutic preparation for the subject on the basis of an analysis result of the analysis unit.
(2)
   The information processing device according to the item (1), in which the characteristic amount relating to the activity of the mitochondria is specified by a machine learning algorithm.
(3)
   The information processing device according to the item (2), in which the machine learning algorithm is generated by deep learning using an image including the mitochondria of the immune cells collected from a patient for whom the immunotherapeutic preparation is efficacious and an image including the mitochondria of the immune cells collected from a patient for whom the immunotherapeutic preparation is not efficacious.
(4)
   The information processing device according to the item (1), in which the characteristic amount relating to the activity of the mitochondria is a morphological or dynamic characteristic amount of the mitochondria.
(5)
   The information processing device according to the item (1), in which the characteristic amount relating to the activity of the mitochondria is a characteristic amount of fluorescence emitted from the mitochondria.
(6)
   The information processing device according to the item (4), in which the morphological characteristic amount of the mitochondria is one or more of a circularity of the mitochondria, an outer peripheral length thereof, and an occupation area thereof in the immune cells.
(7)
   The information processing device according to the item (4), in which the dynamic characteristic amount of the mitochondria is one or more of a frequency of fluctuation of the mitochondria, a velocity thereof, and an amplitude thereof.
(8)
   The information processing device according to the item (5), in which
   at least one of the immune cells or the mitochondria of the immune cells are labeled with a fluorescent molecule, and
   the characteristic amount of the fluorescence is specified on the basis of information regarding a saturation of the fluorescence, a brightness thereof, or a plane distribution thereof.
(9)
   The information processing device according to the item (1), in which the analysis unit analyzes a predetermined morphological or dynamic characteristic amount specified from the image, or a characteristic amount of fluorescence.
(10)
   The information processing device according to any one of the items (1) to (9), in which the output unit evaluates the activity of the mitochondria on the basis of an analysis result of the analysis unit, and outputs information regarding efficacy of the immunotherapeutic preparation for the subject.
(11)
   The information processing device according to any one of the items (1) to (9), in which the output unit determines whether the mitochondria are active or inactive on the basis of an analysis result of the analysis unit, and outputs information regarding efficacy of the immunotherapeutic preparation for the subject on the basis of the ratio of the mitochondria determined to be active.
(12)
   The information processing device according to any one of the items (1) to (9), in which the output unit determines whether the mitochondria are active or inactive on the basis of an analysis result of the analysis unit, and outputs information regarding efficacy of the immunotherapeutic preparation for the subject on the basis of the amount of the mitochondria determined to be active.
(13)
   The information processing device according to any one of the items (1) to (12), in which the information regarding the efficacy indicates efficacy of the immunotherapeutic preparation for the subject.
(14)
   The information processing device according to any one of the items (1) to (13), in which the immunotherapeutic preparation is an immune checkpoint inhibitor.
(15)
   The information processing device according to the item (14), in which the immune checkpoint inhibitor is a PD-1 inhibitor.
(16)
   The information processing device according to any one of the items (1) to (15), in which there is a plurality of types of the immune cells.
(17)
   The information processing device according to any one of the items (1) to (16), in which the immune cells include lymphocytes.
(18)
   The information processing device according to the item (17), in which the lymphocytes include CD8 + T cells.
(19)
   The information processing device according to any one of the items (1) to (18), in which the subject is a cancer patient to whom the immunotherapeutic preparation has been administered.
(20)
   An information processing method for causing an arithmetic processing device
   to acquire an image including mitochondria of immune cells collected from a subject,
   to analyze a characteristic amount relating to activity of the mitochondria specified from the acquired image, and
   to output information regarding efficacy of an immunotherapeutic preparation for the subject on the basis of a result of the analysis.
(21)
   A program that causes a computer to function as
   an image acquiring unit that acquires an image including mitochondria of immune cells collected from a subject,
   an analysis unit that analyzes a characteristic amount relating to activity of the mitochondria specified from the acquired image, and
   an output unit that outputs information regarding efficacy of an immunotherapeutic preparation for the subject on the basis of an analysis result of the analysis unit.

### REFERENCE SIGNS LIST

- 10: Measuring device
- 20: Information processing device
- 30, 40: Terminal device
- 100: System
- 202: Image acquiring unit
- 204: Analysis unit
- 206: Output unit

## Claims

1. An information processing device comprising:
an image acquiring unit that acquires an image including mitochondria of immune cells collected from a subject;
an analysis unit that analyzes a characteristic amount relating to activity of the mitochondria specified from the acquired image; and
an output unit that outputs information regarding efficacy of an immunotherapeutic preparation for the subject on a basis of an analysis result of the analysis unit.

2. The information processing device according to claim 1, wherein the characteristic amount relating to the activity of the mitochondria is specified by a machine learning algorithm.

3. The information processing device according to claim 2, wherein the machine learning algorithm is generated by deep learning using an image including the mitochondria of the immune cells collected from a patient for whom the immunotherapeutic preparation is efficacious and an image including the mitochondria of the immune cells collected from a patient for whom the immunotherapeutic preparation is not efficacious.

4. The information processing device according to claim 1, wherein the characteristic amount relating to the activity of the mitochondria is a morphological or dynamic characteristic amount of the mitochondria.

5. The information processing device according to claim 1, wherein the characteristic amount relating to the activity of the mitochondria is a characteristic amount of fluorescence emitted from the mitochondria.

6. The information processing device according to claim 4, wherein the morphological characteristic amount of the mitochondria is one or more of a circularity of the mitochondria, an outer peripheral length thereof, and an occupation area thereof in the immune cells.

7. The information processing device according to claim 4, wherein the dynamic characteristic amount of the mitochondria is one or more of a frequency of fluctuation of the mitochondria, a velocity thereof, and an amplitude thereof.

8. The information processing device according to claim 5, wherein
at least one of the immune cells or the mitochondria of the immune cells are labeled with a fluorescent molecule, and
the characteristic amount of the fluorescence is specified on a basis of information regarding a saturation of the fluorescence, a brightness thereof, or a plane distribution thereof.

9. The information processing device according to claim 1, wherein the analysis unit analyzes a predetermined morphological or dynamic characteristic amount specified from the image, or a characteristic amount of fluorescence.

10. The information processing device according to claim 1, wherein the output unit evaluates the activity of the mitochondria on a basis of an analysis result of the analysis unit, and outputs information regarding efficacy of the immunotherapeutic preparation for the subject.

11. The information processing device according to claim 1, wherein the output unit determines whether the mitochondria are active or inactive on a basis of an analysis result of the analysis unit, and outputs information regarding efficacy of the immunotherapeutic preparation for the subject on a basis of a ratio of the mitochondria determined to be active.

12. The information processing device according to claim 1, wherein the output unit determines whether the mitochondria are active or inactive on a basis of an analysis result of the analysis unit, and outputs information regarding efficacy of the immunotherapeutic preparation for the subject on a basis of an amount of the mitochondria determined to be active.

13. The information processing device according to claim 1, wherein the information regarding the efficacy indicates efficacy of the immunotherapeutic preparation for the subject.

14. The information processing device according to claim 1, wherein the immunotherapeutic preparation is an immune checkpoint inhibitor.

15. The information processing device according to claim 14, wherein the immune checkpoint inhibitor is a PD-1 inhibitor.

16. The information processing device according to claim 1, wherein there is a plurality of types of the immune cells.

17. The information processing device according to claim 1, wherein the immune cells include lymphocytes.

18. The information processing device according to claim 17, wherein the lymphocytes include CD8 + T cells.

19. The information processing device according to claim 1, wherein the subject is a cancer patient to whom the immunotherapeutic preparation has been administered.

20. An information processing method for causing an arithmetic processing device
to acquire an image including mitochondria of immune cells collected from a subject,
to analyze a characteristic amount relating to activity of the mitochondria specified from the acquired image, and
to output information regarding efficacy of an immunotherapeutic preparation for the subject on a basis of a result of the analysis.

21. A program that causes a computer to function as
an image acquiring unit that acquires an image including mitochondria of immune cells collected from a subject,
an analysis unit that analyzes a characteristic amount relating to activity of the mitochondria specified from the acquired image, and
an output unit that outputs information regarding efficacy of an immunotherapeutic preparation for the subject on a basis of an analysis result of the analysis unit.
